# EUROPEAN PATENT SPECIFICATION

(11) **EP 2 628 448 B1**
(45) Date of publication and mention of the grant of the patent: **04.11.2015**
(21) Application number: 13155191.3
(22) Date of filing: 14.02.2013
(51) Int. Cl.: A61B 8/00, G06F 1/16

(54) **Portable ultrasonic diagnostic apparatus**
Tragbare Ultraschalldiagnosevorrichtung
Appareil de diagnostic ultrasonique portable

(30) Priority: 17.02.2012 KR 20120016552
(43) Date of publication of application: 21.08.2013
(73) Proprietor: Samsung Medison Co., Ltd., Gangwon-do 250-870 (KR)
(72) Inventor: Kim, Soon Deok, Gyeonggi-do (KR); Song, Jung Sik, Seoul (KR)
(74) Representative: Schmid, Wolfgang

(56) References cited:
- EP-A1- 2 138 100
- WO-A1-00/66003
- CN-Y- 201 101 539
- US-A- 5 437 367
- US-A1- 2003 141 205
- US-A1- 2004 152 982

## Description

### 1. Technical Field

Present application relates to a portable ultrasonic diagnostic apparatus that is easily carried by a user.

### 2. Background

Generally, an ultrasonic diagnostic apparatus is an apparatus that emits ultrasonic waves from the surface of the body of an object to an internal body region to be diagnosed and acquires tomograms of soft tissue and images of blood flow via the ultrasonic waves reflected from the body of the object.

A portable ultrasonic diagnostic apparatus includes a display unit including a display provided at the front surface thereof, a probe connected to the display unit and transmitting and receiving ultrasonic waves, a body accommodating the display unit and a control unit to control operation of the apparatus.

The display unit needs to be placed on a stand, in order to use such a portable ultrasonic diagnostic apparatus more conveniently. However, it is not easy to carry the stand due to large volume thereof. A need exists for a way to easily hold a display unit to the portable diagnostic apparatus.

WO 00/66003 A1 discloses a mobile ultrasound diagnostic instrument including a self-powered ultrasound console having electronics for driving a transducer array, processing reflected ultrasound waves, and a visual display for processed ultrasound waves. A docking stand is provided for the console that includes a sleeve for slide receiving the console of the instrument, the sleeve being configured to expose the visual display and manual controls of the console.

Said document represents the closest prior art disclosing the preamble of claim 1.

EP 2 138 100 A1 discloses a portable ultrasonic diagnostic apparatus. The ultrasonic diagnostic apparatus includes a body part, a handle coupled to the body part and having a curved shape, and a display unit coupled to the handle. The handle has a curved shape to improve close contact feelings with an operator's palm.

US 2004/152982 A1 describes a modular diagnostic ultrasound apparatus comprising a core unit, system electronics and an I/O port. The core unit comprises a housing and a system electronics package within the housing. The system electronics has one or more concatenated filters, includes a front end transmit/receive circuit, a processor, a back end circuit for scan conversion, a system clock and a programmable system memory device.

### SUMMARY

An aspect of the present disclosure encompasses a portable ultrasonic diagnostic apparatus that is convenient to use.

Additional aspects of the disclosure will be set forth in part in the description which follows and, in part, will be obvious from the description, or may be learned by practice of the invention.

One aspect of the present disclosure relates to a portable ultrasonic diagnostic apparatus according to claim 1. The portable ultrasonic diagnostic apparatus further includes wheels protruding downward from the body and supported by the ground

The portable ultrasonic diagnostic apparatus may further include a probe connected to the body or the display unit, transmitting ultrasonic waves to an object, and receiving ultrasonic waves from the object.

The body includes a case defining an appearance of the body and the display unit may be accommodated by the case.

The body may include a control unit provided in the case.

The handle may include a handle portion for gripping and at least one elevation portion that is vertically collapsible to the body and connected to the handle portion via an upper end, and the display unit may be detachably mounted on the elevation portion.

The at least one elevation portion may include a pair of elevation portions extending in parallel to each other and connected to the handle portion via upper ends.

The portable ultrasonic diagnostic apparatus may further include a hinge assembly disposed between the display unit and the elevation portion to allow the display unit to be rotatably coupled to the handle.

The hinge assembly may include a first hinge bracket mounted on the rear surface of the display unit, a second hinge bracket coupled to the handle, and a hinge member coupled to the first hinge bracket so as to be rotatable upward or downward and coupled to the second hinge bracket so as to be rotatable clockwise or counterclockwise.

The second hinge bracket may include an engagement protrusion protruding backward and coupled to the elevation portion, and the elevation portion may have an engagement hole to which the engagement protrusion is coupled.

The case includes a probe holder that rotatably protrudes from the case and holds the probe.

The probe holder may have one end rotatably attached to a side of the case such that the probe holder is rotatably collapsed to form a surface of the case, and protrudes rotatably from the case as the probe holder rotates.

The display unit may include a display disposed at the front surface of the display unit and displaying an image.

The display may include a touch-screen.

The body may further include an auxiliary handle disposed at one side of the body.

The case may have a recessed platform on an upper surface.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a perspective view illustrating a portable ultrasonic diagnostic apparatus according to an embodiment of the present disclosure;
FIG. 2 is a perspective view illustrating a portable ultrasonic diagnostic apparatus according to an embodiment of the present disclosure in use;
FIG. 3 is a perspective view illustrating a display unit of a portable ultrasonic diagnostic apparatus according to an embodiment of the present disclosure mounted on a handle; and
FIG. 4 is a perspective view illustrating a display unit of a portable ultrasonic diagnostic apparatus according to another embodiment of the present disclosure mounted on a handle.

### DETAILED DESCRIPTION

Reference will now be made in detail to the embodiments of the present disclosure, examples of which are illustrated in the accompanying drawings, wherein like reference numerals refer to like elements throughout.

Hereinafter, a portable ultrasonic diagnostic apparatus according to an embodiment of the present disclosure will be described in detail.

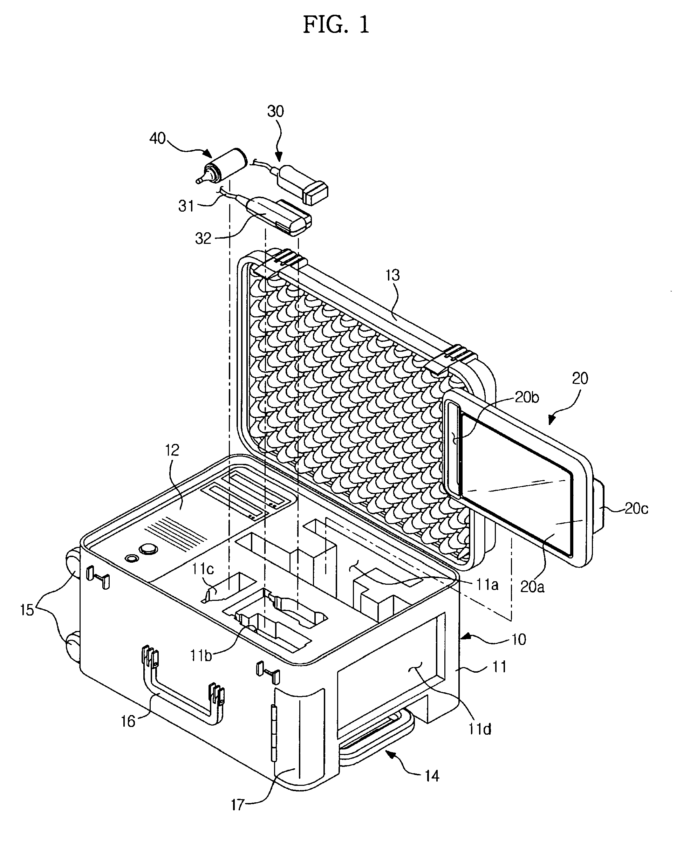

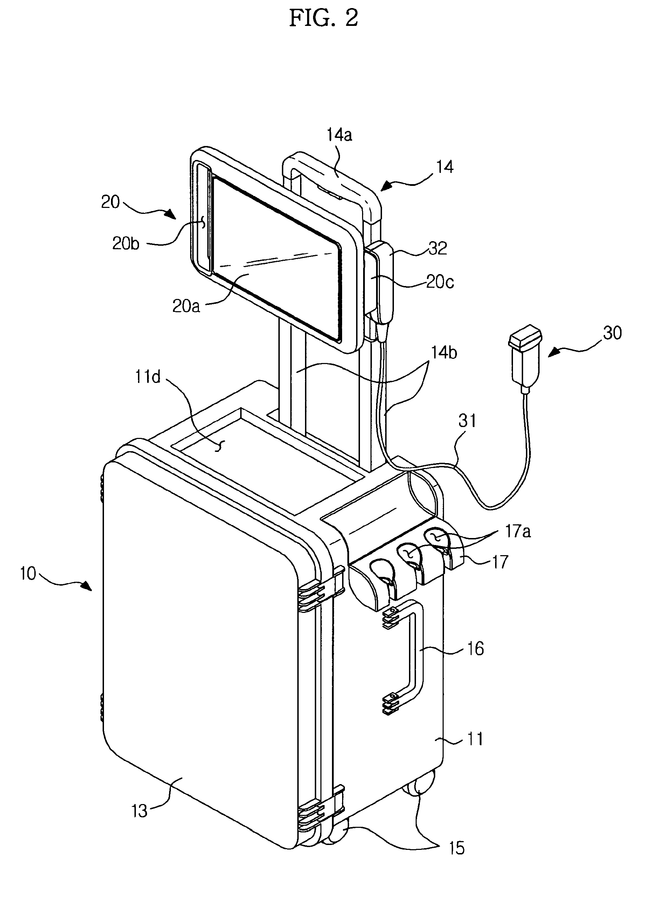

Referring to FIGS. 1 and 2, the portable ultrasonic diagnostic apparatus includes a body 10, a display unit 20, and a probe 30. The display unit 20 includes a display 20a disposed at the front surface thereof and displaying an image such as diagnosis results. The probe 30 is connected to the display unit 20, transmits ultrasonic waves to an object to be diagnosed, and receives ultrasonic waves reflected by the object.

Referring to FIGS. 1 and 2, the body 10 includes a case 11 defining an appearance thereof, and a control unit 12 disposed in the case 11 and controlling operation of the portable ultrasonic diagnostic apparatus. The display unit 20 and the probe 30 may be accommodated in the case 11.

The display 20a is provided at the front surface of the display unit 20 as described above. According to the present embodiment, the display 20a is a touch-screen such that a user may control operation of the portable ultrasonic diagnostic apparatus via the touch of the display 20a. In addition, the display unit 20 has a handle groove 20b at a side thereof to allow the user to easily hold the display unit 20 and includes a terminal 20c disposed at the rear surface of the display unit 20 and connected to the probe 30.

The probe 30 is detachably connected to the display unit 20. To this end, the probe 30 includes a cable 31 having a predetermined length and a connector 32 disposed at one end of the cable 31 to connect to the terminal 20c.

The case 11, one side of which is open, has a plurality of accommodation portions 11a, 11b, and 11c to accommodate the display unit 20, the probe 30, and the like. A cover 13 has one end rotatably coupled to one side of the case 11, and opens and closes the accommodation portions 11 a, 11 b, and 11 c as the cover 13 rotates with respect to the case 11. The case 11 includes a handle 14 that is movably disposed on one side of the case 11, and retracts downward into and protrudes upward from the case 11 to allow the user to pull the body 10, a plurality of wheels 15 that protrude downward from the case 11 and are supported by the ground, and an auxiliary handle 16 that is disposed at one side of the case 11 to allow the user to carry the body 10.

The case 11 may further accommodate a gel container 40 containing ultrasound gel used in ultrasonic diagnosis and any other accessories in addition to the display unit 20 and the probe 30. The accommodation portions 11a, 11b, and 11c may include a first accommodation portion 11a to accommodate the display unit 20, a second accommodation portion 11b to accommodate the probe 30, a third accommodation portion 11c to accommodate the gel container 40, and the like.

The case 11 includes a recessed platform 11d on which accessories used for ultrasonic diagnosis such as the gel container 40 are placed, and a probe holder 17 protruding from one side of the case 11 and housing the probe 30. The probe holder 17 has one end rotatably attached to the case 11, and restores into or projects away from the case 11 as the probe holder 17 rotates with respect to the case 11. The probe holder 17 has a plurality of probe holding grooves 17a in which the probe 30 is held.

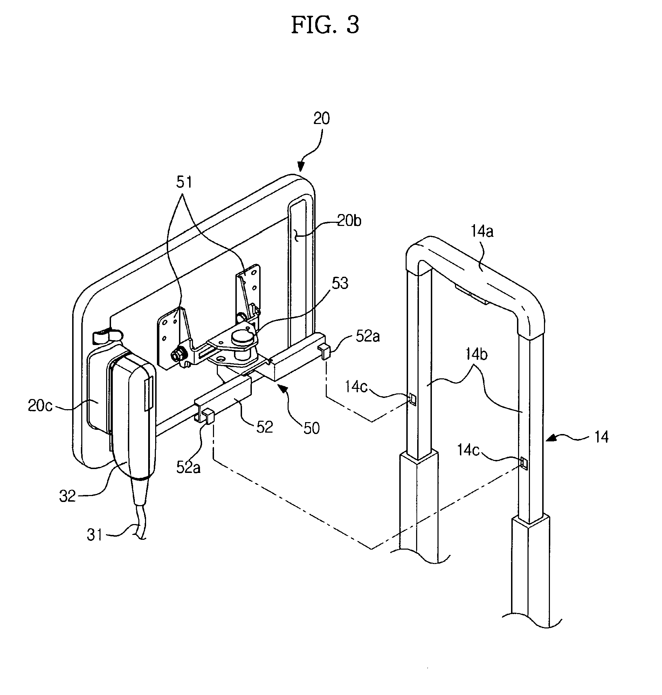

Referring to FIG. 3, the handle 14 installed to the side of the case 11 is vertically movable to allow the user to selectively adjust the length of a protruding portion of the handle 14. The handle 14 includes a handle portion 14a to allow the user to grip the handle 14 and pull the case 11 and an elevation portion 14b vertically movable along the side of the case 11 and protruding upward from the case 11. The handle portion 14a is connected to an upper end of the elevation portion 14b. According to the present example, the elevation portion 14b includes a pair of elevation portions 14b disposed in parallel to each other, and upper ends of both elevation portions 14b are connected to both ends of the handle portion 14a.

The display unit 20 is detachably mounted on the handle 14 provided to the body 10, and thus the body 10 may be used as a stand to support the display unit 20. That is, since the display unit 20 can be attached to the elevation portions 14b of the handle 14 protruding upward from the case 11, the display unit 20 may be kept at a predetermined height. In this regard, the height of the display unit 20 may be adjusted by moving the elevation portions 14b of the handle 14 to which the display unit 20 is mounted upward or downward since the handle 14 is vertically movable along the one side of the case 11.

Since the display unit 20 is rotatably installed with respect to the handle 14, the display unit 20 may be rotated within a predetermined range. To this end, a hinge assembly 50 is disposed between the display unit 20 and the handle 14 as shown in FIG. 3, so that the display unit 20 is rotatably installed to the handle 14 by the hinge assembly 50.

The hinge assembly 50 includes a pair of first hinge brackets 51 installed at opposite sides of the rear surface of the display unit 20, a pair of second hinge brackets 52 detachably attached to the elevation portions 14b, and a hinge member 53 having both ends coupled to the first hinge brackets 51 so as to be rotatable upward or downward and a central portion coupled to the second hinge brackets so as to be rotatable clockwise or counterclockwise. Accordingly, the display unit 20 may be rotatably attached to the elevation portions 14b of the handle 14 via the hinge assembly 50 so as to be rotatable upward or downward or clockwise or counterclockwise.

In order to detachably install the hinge assembly 50 to the elevation portions 14b, each of the second hinge brackets 52 has an engagement protrusion 52a, which protrudes backward, and is coupled to each of the elevation portions 14b, and each of the elevation portions 14b has an engagement hole 14c to which the engagement protrusion 52a is coupled. Thus, the hinge assembly 50 and the display unit 20 are mounted on the handle 14 by coupling the engagement protrusion 52a of the hinge assembly 50 to the engagement hole 14c of the elevation portion 14b. The hinge assembly 50 and the display unit 20 are separated from the handle 14 by detaching the engagement protrusion 52a from the engagement hole 14c.

According to the present embodiment, the display unit 20 is fixed to the hinge assembly 50, and the hinge assembly 50 is coupled to the handle 14 via the engagement protrusion 52a formed at the rear surface of the display unit 20. However, the disclosure is not limited thereto, and the display unit 20 may also be directly coupled to the handle 14 by forming the engagement protrusion 52a at the display unit 20.

In addition, the display 20a of the display unit 20 applied to the portable ultrasonic diagnostic apparatus is a touch-screen, and the display unit 20 is controlled via the display 20a according to the present embodiment. However, the disclosure is not limited thereto, and the display unit 20 may also be controlled by a control panel (not shown) separately formed from the display unit 20.

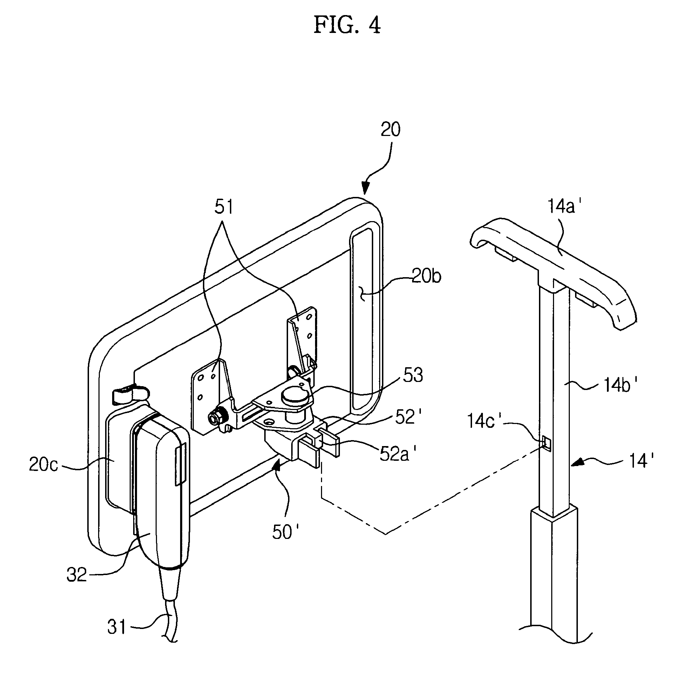

In addition, according to the present embodiment, the handle 14 includes a pair of elevation portions 14b. However, the disclosure is not limited thereto, and the handle 14 may include an elevation portion 14b' and a handle portion 14a' connected to the elevation portion 14b' at the center as illustrated in FIG. 4.

According to the present embodiment, the control unit 12 is installed in the body 10. However, the disclosure is not limited thereto, and the control unit 12 may be integrally formed with the display unit 20.

According to the present embodiment, the probe 30 is connected to the display unit 20. However, the disclosure is not limited thereto, and the probe 30 may be connected to the body 10.

As is apparent from the above description, the portable ultrasonic diagnostic apparatus includes the display unit detachably mounted on the handle of the body. Since the handle is used as a stand supporting the display unit, the portable ultrasonic diagnostic apparatus may become more convenient to use.

## Claims

1. A portable ultrasonic diagnostic apparatus comprising:
a body (10) comprising a case (11) forming an external appearance of the body (10);
a handle (14) installed at a side of the body (10) and configured for protruding from or retracting into the body (10);
a display unit (20) detachably mounted on the handle (14); and
a probe (30) configured for transmitting and receiving ultrasonic waves to and from an object, respectively, **characterised in that** the probe (30) can be accommodated in the case (11),
and the case (11) includes a probe holder (17) that is rotatably protrusible from the case (11) to hold the probe (30).

2. The portable ultrasonic diagnostic apparatus according to claim 1, wherein the probe (30) is connected to one of the body (10) and the display unit (20).

3. The portable ultrasonic diagnostic apparatus according to claim 1, wherein the display unit (20) can be accommodated the case (11).

4. The portable ultrasonic diagnostic apparatus according to claim 1, wherein the body (10) further comprises a control unit (12) provided in the case (11).

5. The portable ultrasonic diagnostic apparatus according to claim 1, wherein:
the handle (14) comprises a handle portion (14a) for gripping and at least one elevation portion (14b) that is vertically collapsable to the body (10) and connected to the handle portion (14a) via an upper end; and
the display unit (20) is detachably mounted on the elevation portion (14b).

6. The portable ultrasonic diagnostic apparatus according to claim 5, wherein the at least one elevation portion (14b) comprises a pair of elevation portions (14b) extending in parallel to each other and connected to the handle portion (14a) via upper ends.

7. The portable ultrasonic diagnostic apparatus according to claim 6, further comprising a hinge assembly (50) disposed between the display unit (20) and the elevation portion (14b) to allow the display unit (20) to be rotatably coupled to the handle (14).

8. The portable ultrasonic diagnostic apparatus according to claim 7, wherein the hinge assembly (50) comprises a first hinge bracket (51) mounted on the rear surface of the display unit (20), a second hinge bracket (52) coupled to the handle (14), and a hinge member (53) coupled to the first hinge bracket (51) so as to be rotatable upward or downward and coupled to the second hinge bracket (52) so as to be rotatable clockwise or counterclockwise.

9. The portable ultrasonic diagnostic apparatus according to claim 8, wherein:
the second hinge bracket (52) comprises an engagement protrusion (52a) protruding backward and coupled to the elevation portion (14b); and
the elevation portion (14b) has an engagement hole (14c) to which the engagement protrusion (52a) is coupled.

10. The portable ultrasonic diagnostic apparatus according to claim 1, wherein the probe holder (17) has one end rotatably attached to a side of the case (11) such that the probe holder (17) is rotably collapsed to form a surface of the case (11), and protrudes rotatably from the case (11) as the probe holder (17) rotates.

11. The portable ultrasonic diagnostic apparatus according to claim 1, wherein the display unit (20) comprises a display (20a) disposed at the front surface of the display unit (20) for displaying an image.

12. The portable ultrasonic diagnostic apparatus according to claim 11, wherein the display (20a) comprises a touch-screen.

13. The portable ultrasonic diagnostic apparatus according to claim 1, wherein the body (10) further comprises an auxiliary handle (16) disposed at one side of the body (10).

14. The portable ultrasonic diagnostic apparatus according to claim 1, wherein the case (11) further comprises a recessed platform (11 d) on an upper surface of the case (11).

## Patentansprüche

1. Tragbare Ultraschalldiagnosevorrichtung, welche Folgendes aufweist:
einen Körper (10) mit einem Gehäuse (11), der ein äußeres Erscheinungsbild des Körpers (10) bildet;
einen Handgriff (14), der an einer Seite des Körpers (10) angeordnet und dafür vorgesehen ist, über den Körper (10) überzustehen oder in denselben eingezogen zu sein;
eine Anzeigeeinheit (20), die abnehmbar an dem Handgriff (14) angebracht ist; und
eine Sonde bzw. einen Prüfkopf (30), der dafür vorgesehen ist, Ultraschallwellen zu und von einem Objekt zu übertragen und zu empfangen,
**dadurch gekennzeichnet, dass**
der Prüfkopf (30) in dem Gehäuse (11) angeordnet sein kann,
und das Gehäuse (11) einen Prüfkopfhalter (17) aufweist, der auf drehbare Art und Weise von dem Gehäuse (11) ausfahrbar ist, um den Prüfkopf (30) zu halten.

2. Tragbare Ultraschalldiagnosevorrichtung nach Anspruch 1, wobei der Prüfkopf (30) entweder mit dem Körper (10) oder der Anzeigeeinheit (20) verbunden ist.

3. Tragbare Ultraschalldiagnosevorrichtung nach Anspruch 1, wobei die Anzeigeeinheit (20) in dem Gehäuse (11) angeordnet sein kann.

4. Tragbare Ultraschalldiagnosevorrichtung nach Anspruch 1, wobei der Körper (10) des Weiteren eine Steuereinheit (12) aufweist, die in dem Gehäuse (11) angeordnet ist.

5. Tragbare Ultraschalldiagnosevorrichtung nach Anspruch 1, wobei:
der Handgriff (14) einen Handgriffabschnitt (14a) zum Greifen und wenigstens einen Verlängerungsabschnitt (14b) aufweist, der vertikal in den Körper (10) einschiebbar und über ein oberes Ende mit dem Handgriffabschnitt (14a) verbunden ist; und
die Anzeigeeinheit (20) abnehmbar an dem Verlängerungsabschnitt (14b) angebracht ist.

6. Tragbare Ultraschalldiagnosevorrichtung nach Anspruch 5, wobei der wenigstens eine Verlängerungsabschnitt (14b) ein paar Verlängerungsabschnitte (14b) aufweist, die sich parallel zueinander erstrecken und über obere Enden mit dem Handgriffabschnitt (14a) verbunden sind.

7. Tragbare Ultraschalldiagnosevorrichtung nach Anspruch 6, welche des Weiteren eine Gelenkanordnung (50) aufweist, die zwischen der Anzeigeeinheit (20) und dem Verlängerungsabschnitt (14b) angeordnet ist, um zu ermöglichen, dass die Anzeigeeinheit (20) drehbar mit dem Handgriff (14) verbunden ist.

8. Tragbare Ultraschalldiagnosevorrichtung nach Anspruch 7, wobei die Gelenkanordnung (50) eine erste Gelenkhalterung (51), die auf der hinteren Fläche der Anzeigeeinheit (20) montiert ist, eine zweite Gelenkhalterung (52), die mit dem Handgriff (14) verbunden ist, und ein Gelenkelement (53) aufweist, das mit der ersten Gelenkhalterung (51) verbunden ist, um nach oben oder nach unten drehbar zu sein, und mit der zweiten Gelenkhalterung (52) verbunden ist, um im Uhrzeigersinn oder entgegen dem Uhrzeigersinn drehbar zu sein.

9. Tragbare Ultraschalldiagnosevorrichtung nach Anspruch 8, wobei:
die zweite Gelenkhalterung (52) einen Eingriffsvorsprung (52a) aufweist, der sich nach hinten erstreckt und mit dem Verlängerungsabschnitt (14b) verbunden ist; und
der Verlängerungsabschnitt (14b) eine Eingriffsbohrung (14c) aufweist, mit welcher der Eingriffsvorsprung (52a) verbunden ist.

10. Tragbare Ultraschalldiagnosevorrichtung nach Anspruch 1, wobei ein Ende des Prüfkopfhalters (17) derart drehbar an einer Seite des Gehäuses (11) angebracht ist, dass der Prüfkopfhalter (17) drehbar zusammengeklappt werden kann, um eine Oberfläche des Gehäuses (11) zu bilden, und drehbar über das Gehäuse (11) übersteht, wenn der Prüfkopfhalter (17) rotiert wird.

11. Tragbare Ultraschalldiagnosevorrichtung nach Anspruch 1, wobei die Anzeigeeinheit (20) eine Anzeige (20a) aufweist, die an der vorderen Fläche der Anzeigeeinheit (20) angeordnet ist, um ein Bild anzuzeigen.

12. Tragbare Ultraschalldiagnosevorrichtung nach Anspruch 11, wobei die Anzeige (20a) einen Touchscreen aufweist.

13. Tragbare Ultraschalldiagnosevorrichtung nach Anspruch 1, wobei der Körper (10) des Weiteren einen Hilfshandgriff (16) aufweist, der an einer Seite des Körpers (10) angeordnet ist.

14. Tragbare Ultraschalldiagnosevorrichtung nach Anspruch 1, wobei das Gehäuse (11) des Weiteren eine vertiefte Ebene (11 d) auf einer oberen Fläche des Gehäuses (11) aufweist.

## Revendications

1. Appareil de diagnostic ultrasonique portable comportant :
un corps (10) comportant un boîtier (11) définissant une forme extérieure du corps (10) ;
une poignée (14) montée sur un côté du corps (10) et configurée pour être en protubérance ou se rétracter par rapport au corps (10) ;
une unité d'affichage (20) détachable, montée sur la poignée (14) ; et une sonde (30) configurée pour transmettre et recevoir des ondes ultrasoniques à destination et en provenance d'un objet respectivement, **caractérisé en ce que** la sonde (30) peut être logée dans le boîtier (11),
et le boîtier (11) comporte un support (17) de sonde qui est protubérant en rotation hors du boîtier (11) pour maintenir la sonde (30).

2. Appareil de diagnostic ultrasonique portable selon la revendication 1, dans lequel la sonde (30) est connectée soit au boîtier (10) soit à l'unité d'affichage (20).

3. Appareil de diagnostic ultrasonique portable selon la revendication 1, dans lequel l'unité d'affichage (20) peut être logée dans le boîtier (11).

4. Appareil de diagnostic ultrasonique portable selon la revendication 1, dans lequel le corps (10) comporte en outre une unité de commande (12) logée dans le boîtier (11).

5. Appareil de diagnostic ultrasonique portable selon la revendication 1, dans lequel :
la poignée (14) comporte une portion de poignée (14a) à saisir et au moins une portion en élévation (14b) qui est extensible verticalement par rapport au corps (10) et couplée à la portion de poignée (14a) par une extrémité supérieure ; et
l'unité d'affichage (20) est montée de façon détachable sur la portion en élévation (14b).

6. Appareil de diagnostic ultrasonique portable selon la revendication 5, dans lequel au moins une portion en élévation (14b) comporte une paire de portions en élévation (14b) qui s'étendent parallèlement l'une par rapport à l'autre et sont connectées à la portion de poignée (14a) par les extrémités supérieures.

7. Appareil de diagnostic ultrasonique portable selon la revendication 6, comportant en outre un assemblage articulé (50) disposé entre l'unité d'affichage (20) et la portion en élévation (14b) pour permettre à l'unité d'affichage (20) d'être couplée en rotation à la poignée (14).

8. Appareil de diagnostic ultrasonique portable selon la revendication 7, dans lequel l'assemblage articulé (50) comporte un premier support de charnière (51) monté sur la surface arrière de l'unité d'affichage (20), un second support de charnière (52) couplé à la poignée (14), et un élément d'articulation (53) couplé au premier support de charnière (51) de manière à être rotatif vers le haut et vers le bas et couplé au second support de charnière (52) de manière à être rotatif dans le sens ou à contresens des aiguilles d'une montre.

9. Appareil de diagnostic ultrasonique portable selon la revendication 8, dans lequel :
le second support de charnière (52) comporte une protubérance de butée (52a) qui est proéminente vers l'arrière et couplée à la portion en élévation (14b) ; et
la portion en élévation (14b) comporte une ouverture d'engagement (14c) avec laquelle la protubérance de butée (52a) est couplée.

10. Appareil de diagnostic ultrasonique portable selon la revendication 1, dans lequel le support de sonde (17) a une extrémité fixée en rotation d'un côté du boîtier (11) de telle manière que le support de sonde (17) soit replié en rotation pour former une surface du boîtier (11) et est proéminent en rotation à partir du boîtier (11) lorsque le support de sonde (17) tourne.

11. Appareil de diagnostic ultrasonique portable selon la revendication 1, dans lequel l'unité d'affichage (20) comporte un écran (20a) disposé sur la surface frontale de l'unité d'affichage (20) pour afficher une image.

12. Appareil de diagnostic ultrasonique portable selon la revendication 11, dans lequel l'écran (20a) comporte un écran tactile.

13. Appareil de diagnostic ultrasonique portable selon la revendication 1, dans lequel le corps (10) comporte en outre une poignée auxiliaire (16) disposée sur un côté du corps (10).

14. Appareil de diagnostic ultrasonique portable selon la revendication 1, dans lequel le boîtier (11) comporte en outre une plateforme rétractée (11 d) sur une surface supérieure du boîtier (11).
